# EUROPÄISCHE PATENTSCHRIFT

(11) **EP 2 523 691 B1**
(45) Veröffentlichungstag und Bekanntmachung des Hinweises auf die Patenterteilung: **01.10.2014**
(21) Anmeldenummer: 11700182.6
(22) Anmeldetag: 14.01.2011
(51) Int. Cl.: A61K 47/48, A61K 9/00, A61K 31/08, A61P 23/00, C08B 37/16, B82Y 5/00

(54) **FLURANKOMPLEX**
HALOGENATED ETHER COMPLEX
COMPLEXE D'ÉTHER HALOGÉNÉ

(30) Priorität: 14.01.2010 EP 10150786
(43) Veröffentlichungstag der Anmeldung: 21.11.2012
(73) Patentinhaber: SapioTec GmbH, 97082 Würzburg (DE)
(72) Erfinder: ROEWER, Norbert, 97082 Würzburg (DE); BROSCHEIT, Jens, 97209 Würzburg (DE)
(74) Vertreter: Glawe, Delfs, Moll
(86) Internationale Anmeldenummer: PCT/EP2011/050428
(87) Internationale Veröffentlichungsnummer: WO 2011/086146

(56) Entgegenhaltungen:
- WO-A2-2008/070490
- GB-A- 2 350 297
- US-A- 3 216 897
- US-A- 4 725 442
- VIERNSTEIN H ET AL: "Intravenous anesthesia with isoflurane in the rabbit", PHARMACEUTICAL AND PHARMACOLOGICAL LETTERS, MEDPHARM, SCIENTIFIC PUBL., STUTTGART, DE, Bd. 3, Nr. 5, 1. Januar 1994 (1994-01-01), Seiten 165-168, XP008122434, ISSN: 0939-9488
- BREWSTER ET AL: "Cyclodextrins as pharmaceutical solubilizers", ADVANCED DRUG DELIVERY REVIEWS, ELSEVIER BV, AMSTERDAM, NL LNKD- DOI:10.1016/J.ADDR.2007.05.012, Bd. 59, Nr. 7, 24. August 2007 (2007-08-24), Seiten 645-666, XP022211985, ISSN: 0169-409X

## Beschreibung

Die Erfindung betrifft einen Flurankomplex sowie dessen Formulierung als Anästhetikum.

Flurane sind Arzneistoffe, die in der Anästhetesie zur Aufrechterhaltung oder Einleitung der Narkose eingesetzt werden und im Stand der Technik durch Inhalation aufgenommen werden. Inhalationsanästhetika werden als Gase oder mittels eines Vaporisers verdampfte Flüssigkeiten über eine Atemmaske, eine Larynxmaske oder einen Endotrachealtubus appliziert.

Flurane weisen einen niedrigen Siedepunkt und hohen Dampf auf. Es handelt sich um mehrfach halogenierte Ether. Es handelt sich um lipophile Stoffe, deren anästhetische Wirksamkeit unter anderem mit einer aus der Lipophilie herrührenden unspezifischen Interaktion mit Bestandteilen der Zellmembran erklärt wird.

Viernstein H.; Stumpf C.; Reiter, S. "Intravenous anaesthesia with isoflurane in the rabbit" Pharmaceutical and Pharmacological Letters 1994, 3, 165-168 offenbart eine Lösung beinhaltend einen Komplex aus Hydroxypropyl-β-cyclodextrin (HP-β-CD) und dem Anästhetikum Isofluran zur intravenösen Verabreichung.

Der Erfindung liegt die Aufgabe zugrunde, eine Fluranformulierung zur Verfügung zu stellen, die deren Applikation auf anderem Wege als durch Inhalation gestattet.

Gegenstand der Erfindung ist ein Komplex aus α-Cyclodextrin und einem Fluran, bei dem der Flurangehalt wenigstens 3 Gew.-% des Gesamtgewichtes des Komplexes beträgt.

Die Erfindung hat erkannt, dass sich überraschenderweise Komplexe von Fluranen mit α-Cyclodextrins herstellen lassen, die die Basis für eine Formulierung dieser Anästhetika für die orale oder intravenöse Administration bilden können und einen hohen Flurangehalt von 3 oder mehr Gew.-% aufweisen können.

Cyclodextrine weisen generell eine Toroidform auf und besitzen eine entsprechend geformte Kavität. Die Flurane treten als Gastmolekül in diese Kavität ein, sodass ein in wässriger Lösung formulierbarer Komplex der extrem lipophilen Flurane erhalten wird, der die Flurane am vorgesehenen pharmazeutischen Wirkort zur Verfügung stellen kann.

α-Cyclodextrine weist sechs Glukopyranose-Einheiten auf, von denen jede drei OH-Gruppen trägt, die ggf. substituiert (bspw. methyliert) sein können.

Der Flurangehalt des erfindungsgemäßen Komplexes beträgt bevorzugt wenigstens 5 Gew.-%, weiter vorzugsweise wenigstens 7 Gew.-%, weiter vorzugsweise wenigstens 8 Gew.-%, weiter vorzugsweise 8 bis 12 Gew.-%. Die genannten Werte können beliebig zu erfindungsgemäßen Bereichen kombiniert werden.

Das Fluran ist ein mehrfach fluorierter Ether und bevorzugt ausgewählt aus der Gruppe bestehend aus Sevofluran, Enfluran, Isofluran, Desfluran und Methoxyfluran. Sevofluran ist besonders bevorzugt.

Der Komplex kann erfindungsgemäß einen Wassergehalt (Restwassergehalt) von 5 bis 15 Gew.-%, vorzugsweise 7 bis 13 Gew.-% aufweisen.

Gegenstand der Erfindung ist ferner ein erfindungsgemäßer Komplex zur Verwendung als Medikament.

Ein weiterer Gegenstand der Erfindung ist ein für eine orale und/oder intravenöse Administration formuliertes Anästhetikum, das einen erfindungsgemäßen Komplex enthält.

Gegenstand der Erfindung ist ferner ein Verfahren zur Herstellung eines erfindungsgemäßen Komplexes, mit den Schritten
a) Herstellen einer wässrigen Lösung des α-Cyclodextrins,
b) Zugabe des Flurans zur der wässrigen Lösung,
c) Abtrennen des ausgefällten Komplexes.

Erfindungsgemäß wird im ersten Schritt eine wässrige Lösung des α-Cyclodextrins hergestellt. Bevorzugt handelt es sich um eine vollständige Lösung, bei der kein ungelöstes Cyclodextrin in Suspension verbleibt. Bevorzugte Konzentrationen des α-Cyclodextrins in der wässrigen Lösung sind 5 bis 30 Gew.-%, vorzugsweise 5 bis 20 Gew.-%, weiter vorzugsweise 5 bis 15 Gew.-%. Verwendet man unsubstituiertes α-Cyclodextrins, liegt die Obergrenze bei 14,5 Gew.-% (Löslichkeit von unsubstituierten α-Cyclodextrins in Wasser). Substituierte, insbesondere (Teil)methylierte α-Cyclodextrine können eine höhere Löslichkeit in Wasser aufweisen.

Das Lösen des α-Cylodextrins in Wasser erfolgt bevorzugt bei Raumtemperatur. Zu dem gelösten α-Cyclodextrin wird das Fluran zugegeben. Es bildet sich ein Komplex, der kristallin aus der wässrigen Lösung ausfällt und abgetrennt werden kann.

Im Rahmen der Erfindung ist es bevorzugt, wenn die Zugabe des Flurans in einem Molverhältnis zum α-Cyclodextrin von 1:0,5 bis 1:2, vorzugsweise 1:0,8 bis 1:1,2 erfolgt. Insbesondere kann eine etwa equimolare Zugabe des Flurans im Verhältnis zum Cyclodextrin erfolgen.

Im ausgefällten Komplex findet man ein Molverhältnis von Fluran zum Cyclodextrin etwa im Bereich 1:1,5 bis 1:6. Bevorzugt sind Bereiche von 1:1,5 bis 1:2. Gemessen an dem Molverhältnis von Fluran und Cyclodextrin im ausgefällten Komplex wird also bei der Herstellung bevorzugt ein molarer Überschuss des Flurans verwendet.

Erfindungsgemäß wird bevorzugt nach Zugabe des Flurans gekühlt, beispielsweise auf eine Temperatur von 5 bis 10° C, um das Ausfällen des Komplexes zu begünstigen und damit die Ausbeute zu erhöhen.

Ausführungsbeispiele und Vergleichsbeispiele werden nachfolgend beschrieben.

### 1. Verwendete Materialien

- gereinigtes Wasser (Millipor Q quality water) = α-Cyclodextrin
- β-Cyclodextrin
- 2-Hydroxypropyl-β-cyclodextrin
- Methyl-β-cyclodextrin (RAMEB)
- Sevofluran

Alle Cyclodextrine wurde bezogen von der Firma Cyclo Lab Cyclodextrin Research & Development Laborotory Ltd., Ungarn. Sevofluran wurde bezogen von Abbott GmbH, Wiesbaden.

### 2. Bestimmung des Sevofluran-Gehaltes hergestellter Komplexe

Die Bestimmung des Sevofluran-Gehaltes der Komplexe erfolgte gaschromatografisch. Die Gaschromatografiebedingungen waren wie folgt:

| | |
|---|---|
| Gaschromatograph: | Shimadzu GC-17A |
| Detector: Injector: | Flame ionization detector (FID) |
| | Shimadzu AoC-5000 auto injector |
| *Software:* | Shimadzu Class-VP 7.4 Version |

| | |
|---|---|
| *Gase:* | |
| *Träger:* | Helium (99.999 %) |
| weitere Gase: | Stickstoff (99.999%) |
| | synthetische Luft (99.999%) |
| | Wasserstoff (Whatman Hydrogen generator) |
| | |
| Säule: Rtx624 (30 m x 0.32 mm x 1.8 mm) (Restek) | |

### TEMPERATURPROGRAMM:

| Rate (°C/min) | Temperatur (°C) : | Zeit (min) : |
|---|---|---|
| - | 38 | 4.0 |
| 40 | 220 | 1.5 |

- *Injektortemperatur:*: 220°C
- *Detektortemperatur:*: 220°C
- *Split ratio:*: 100:1
- *Geschwindigkeit:*: 30 cm/s

Das Injektionsprogramm war wie folgt: Nach einer Inkubationszeit von 10 min bei 60°C wurde eine Dampfprobe mit einem Volumen von 250 µl bei 70 °C in den Gaschromatografen injiziert.

### Probenvorbereitung

Vergleichslösungen: 1 ml destilliertes Wasser mit 250 µl DMF wird in die Viale gegeben.

Kalibrierlösungen: Als Stammlösung wird 100 mg Sevofluran in 2 ml Glasflaschen eingewogen und bis zur Markierung mit DMF aufgefüllt.

Verschiedene Mengen dieser Stammlösung (20, 65, 110, 155 und 200 µl) werden jeweils mit DMF auf 200 µl aufgefüllt und mit 1 ml destilliertem Wasser in Head Space Vials (19,5 ml) eingefüllt.

Probenlösung: 50 mg des Probenkomplexes wird in eine Head Space Vial (19,5 ml) gegeben mit 1 ml destilliertem Wasser und 200 µl DMF. Ferner wird 1 ml Mutterlösung (hergestellt aus dem Überstand der Komplexierung von Sevofluran mit Cyclodextrin nach Entfernung des ausgefällten Komplexes) wird mit 200 µl DMF in Head Space Vials (19,5 ml) gegeben.

### Beispiel 1

Dieses Beispiel beschreibt die Herstellung eines α-Cyclodextrin (α-CD) Komplexes von Sevofluran.

In einem Rundkolben werden unter fortlaufendem Rühren bei Raumtemperatur 45,25 g (0,0465 mol) α-CD in 500 ml Wasser gelöst. Nach vollständiger Lösung des α-CD wurden 6 ml (0,0465 mol) Sevofluran bei Raumtemperatur zu der Lösung gegeben. Ein weißes Präzipitat fiel aus und es wurde mit Eiswasser auf 5 bis 10 °C gekühlt. Bei dieser Temperatur wurde 4 h gerührt, dann wurde die Flasche über Nacht im Kühlschrank aufbewahrt. Anschließend wurde der ausgefällte Kristalline Sevofluran/α-CD-Komplex abfiltriert und im Vakuum über Phosphorpentoxid getrocknet.

### Beispiel 2

Dieses Beispiel zeigt den Einfluss der Auswahl des geeigneten Cyclodextrins auf die Bildung des Komplexes. Zu Vergleichszwecken wurden hier β-Cyclodextrin (β-CD), Hydroxypropyl-β-cyclodextrin (HP-β-CD) und RAMEB verwendet. Diese Vergleichsbeispiele sind nicht erfindungsgemäß.

Es wurden jeweils 1 ml einer wässrigen Lösung von 4 Cyclodextrinen hergestellt. Die Konzentration der Cyclodextrine in dieser wässrigen Lösung war wie folgt:
- α-CD:: 10 Gew.-%
- β-CD:: 2 Gew.-%
- HP-β-CD:: 10 Gew.-%
- RAMEB:: 10 Gew.-%

Zu diesen Lösungen wurde Sevofluran im Molverhältnis 1:1 bezogen auf den Molargehalt des jeweiligen Cyclodextrins gegeben.

Bei HP-β-CD und RAMEB ergab sich eine homogene Lösung, ein Komplex wurde nicht ausgefällt.

α-CD und β-CD bildeten ein weißes Präzipitat.

In einem nächsten Schritt wurden jeweils 100 ml wässrige Lösung von α-CD (9,05 Gew.-%) und β-CD (1,78 Gew.-%) hergestellt. Wieder wurde Sevofluran im Molverhältnis 1:1 zugegeben. Die weitere Gewinnung aus gefälltem weißen Präzipitates erfolgte wie in Beispiel 1 beschrieben.

Der Sevoflurangehalt des ausgefällten Komplexes wurde gaschromatografisch bestimmt. Die Ergebnisse sind in der nachfolgenden Tabelle 1 angegeben.

| | α-CD | β-CD |
|---|---|---|
| CD-Konzentration (%) | 9.05 | 1.78 |
| Masse feucht (g) | 10.00 | 0.97 |
| Masse getrocknet (g) | 6.57 | 0.83 |
| Ausbeute (%) | 72.6 | 46.6 |
| Sevoflurangehalt (%) | 9.2 | 1.8 |
| Molverhältnis Sevofluran/CD | 1 : 1.8 | 1 : 8.6 |

Die Tabelle zeigt, dass sich α-CD für eine Bildung eines Komplexes mit hohem Gehalt an Sevofluran eignet, während β-CD nur verhältnismäßig geringe Gehalte von Sevofluran im Komplex ermöglicht.

### Beispiel 3

In diesem Beispiel wurden die Herstellungsbedingungen zur Herstellung eines Sevoflurankomplexes mit α-CD variiert. Die Ergebnisse sind in der nachfolgenden Tabelle 2 dargestellt.

| | 3.1 | 3.2 | 3.3 | 3.4 |
|---|---|---|---|---|
| α-CD (g) | 9.11 | 9.08 | 9.06 | 9.09 |
| Sevofluran (cm³) | 1.2 | 1.2 | 0.6 | 1.2 |
| Molverhältnis Sevofluran/CD | 1 : 1 | 1 : 1 | 1 : 2 | 1 : 1 |
| Wasser (cm³) | 14 | 100 | 100 | 40 |

| Abfiltriertes Produkt | | | | |
|---|---|---|---|---|
| Masse feucht (g) | 17.36 | 10.66 | 6.78 | 19.14 |
| Masse getrocknet (g) | 9.49 | 5.65 | 3.53 | 9.01 |
| Ausbeute (%) | 86.8 | 51.8 | 35.4 | 82.5 |
| Sevoflurangehalt (%) | 3.5 | 10.0 | 11.3 | 5.7 |
| Molverhältnis Sevofluran/CD | 1 : 5.7 | 1 : 1.9 | 1 : 1.7 | 1 : 3.0 |

In Beispiel 3.1 wurde verhältnismäßig wenig Wasser eingesetzt, so dass α-CD nicht vollständig gelöst werden konnte. Man erkennt, dass sich der Sevoflurangehalt des Komplexes bei diesen Herstellungsbedingungen vermindert.

Beispiel 3.4 zeigt einen weiteren Versuch mit einem geringen Wassergehalt. Man erkennt, dass man hier ebenfalls eine hohe Ausbeute erhält, allerdings ist der Sevoflurangehalt des Komplexes mit 5,7 Gew.-% geringer als im Beispiel 1.

Die Beispiele 2.2 und 2.3 arbeiten mit einem höheren Wasseranteil. Hier wird das Molverhältnis Sevofluran/α-Cyclodextrin in der Ausgangslösung unterschiedlich eingestellt (1:1 in Beispiel 3.2, 1:2 in Beispiel 3.3). In beiden Fällen ergibt sich ein hoher Sevoflurangehalt im Komplex. Der Einsatz des Sevoflurans im deutlichen Überschuss im Vergleich zum Molverhältnis im Komplex (Beispiel 2.2) vergrößert die Ausbeute, wie aus der Tabelle ersichtlich.

### Beispiel 4

Aus den vorstehenden Versuchen konnte ermittelt werden, dass eine gute Ausbeute und ein hoher Sevoflurangehalt im Komplex erreicht werden, wenn man eine 9%ige α-Cyclodextrin-Lösung in Wasser herstellt und das Molverhältnis von zugegebenem Sevofluran zu gelöstem α-CD 1:1 beträgt. Die nachfolgende Tabelle 3 zeigt, dass die Herstellung des Komplexes unter diesen Bedingungen verlässlich reproduzierbar ist. In den drei Versuchen ergaben sich weitgehend gleiche bzw. vergleichbare Ergebnisse.

| | 4.1 | 4.2 | 4.3 |
|---|---|---|---|
| α-CD (g) | 45.25 | 45.25 | 45.25 |
| Sevofluran (cm³) | 6.0 | 6.0 | 6.0 |
| Molverhältnis Sevofluran/CD | 1 : 1 | 1 : 1 | 1 : 1 |
| Wasser (cm³) | 500 | 500 | 500 |

| Abfiltriertes Product | | | |
|---|---|---|---|
| Masse feucht (g) | 36.22 | 34.96 | 39.74 |
| Masse getrocknet (g) | 25.07 | 23.36 | 21.22 |
| Ausbeute (%) | 46.1 | 43.0 | 39.0 |
| Wassergehalt (%) | 9.38 | 10.95 | 8.93 |
| Sevoflurangehalt (%) | 9.4 | 8.6 | 8.6 |
| Molverhältnis Sevofluran/CD | 1 : 1.8 | 1 : 1.9 | 1 : 2.0 |

## Patentansprüche

1. Komplex aus α-Cyclodextrin und einem Fluran, **gekennzeichnet durch** einen Gehalt des Flurans von wenigstens 3 Gew.-% am Gesamtgewicht des Komplexes.

2. Komplex nach Anspruch 1, **dadurch gekennzeichnet, dass** der Flurangehalt wenigstens 5 Gew.-%, vorzugsweise wenigstens 7 Gew.-%, weiter vorzugsweise wenigstens 8 Gew.-%, weiter vorzugsweise 8 bis 12 Gew.-% beträgt.

3. Komplex nach Anspruch 1 oder 2, **dadurch gekennzeichnet, dass** das Fluran ausgewählt ist aus der Gruppe bestehend aus Sevofluran, Enfluran, Isofluran, Desfluran und Methoxyfluran.

4. Komplex nach einem der Ansprüche 1 bis 3, **dadurch gekennzeichnet, dass** er einen Wassergehalt von 5 bis 15 Gew.-%, vorzugsweise 7 bis 13 Gew.-% aufweist.

5. Komplex nach einem der Ansprüche 1 bis 4 zur Verwendung als Medikament.

6. Für orale und/oder intravenöse Administration formuliertes Anästhetikum, **dadurch gekennzeichnet, dass** es einen Komplex nach einem der Ansprüche 1 bis 4 aufweist.

7. Verfahren zur Herstellung eines Komplexes nach einem der Ansprüche 1 bis 4, **gekennzeichnet durch** folgende Schritte:
a. Herstellen einer wässrigen Lösung des α-Cyclodextrins,
b. Zugabe des Flurans zur der wässrigen Lösung,
c. Abtrennen des ausgefällten Komplexes.

8. Verfahren nach Anspruch 7, **dadurch gekennzeichnet, dass** die Konzentration des α-Cyclodextrins in der in Schritt a) hergestellten wässrigen Lösung 5 bis 30 Gew.-%, vorzugsweise 5 bis 20 Gew.-%, weiter vorzugsweise 5 bis 15 Gew.-% beträgt.

9. Verfahren nach Anspruch 7 oder 8, **dadurch gekennzeichnet, dass** die Zugabe des Flurans in Schritt b) in einem Molverhältnis zum α-Cyclodextrin von 1:0,5 bis 1:2, vorzugsweise 1:0,8 bis 1:1,2 erfolgt.

10. Verfahren nach einem der Ansprüche 7 bis 9, **dadurch gekennzeichnet, dass** die Zugabe des Flurans in Schritt b) bei Raumtemperatur erfolgt.

11. Verfahren nach einem der Ansprüche 7 bis 10, **dadurch gekennzeichnet, dass** nach der Zugabe des Flurans gekühlt wird, vorzugsweise auf eine Temperatur zwischen 5 und 10°C.

## Claims

1. Complex composed of α-cyclodextrin and a flurane, **characterised by** a flurane content of at least 3 wt.% based on the total weight of the complex.

2. Complex according to claim 1, **characterised in that** the flurane content is at least 5 wt.%, preferably at least 7 wt.%, more preferably at least 8 wt.%, more preferably 8 to 12 wt.%.

3. Complex according to either claim 1 or claim 2, **characterised in that** the flurane is selected from the group consisting of sevoflurane, enflurane, isoflurane, desflurane and methoxyflurane.

4. Complex according to any of claims 1 to 3, **characterised in that** said complex has a water content of 5 to 15 wt.%, preferably 7 to 13 wt.%.

5. Complex according to any of claims 1 to 4 for use as medication.

6. Anaesthetic composition formulated for oral and/or intravenous administration, **characterised in that** said composition comprises a complex according to any of claims 1 to 4.

7. Process for producing a complex according to any of claims 1 to 4, **characterised by** the following steps:
a. preparing an aqueous solution of the α-cyclodextrin,
b. adding the flurane to the aqueous solution,
c. separating the precipitated complex.

8. Process according to claim 7, **characterised in that** the concentration of the α-cyclodextrin in the aqueous solution prepared in step a) is 5 to 30 wt.%, preferably 5 to 20 wt.%, more preferably 5 to 15 wt.%.

9. Process according to either claim 7 or claim 8, **characterised in that** the flurane is added in step b) in a molar ratio relative to the α-cyclodextrin of 1:0.5 to 1:2, preferably 1:0.8 to 1:1.2.

10. Process according to any of claims 7 to 9, **characterised in that** the flurane is added in step b) at room temperature.

11. Process according to any of claims 7 to 10, **characterised in that** the addition of the flurane is followed by cooling said mixture, preferably to a temperature between 5 and 10 °C.

## Revendications

1. Complexe de l'α-cyclodextrine avec un flurane, **caractérisé par** une teneur en flurane d'au moins 3 % en poids du poids total du complexe.

2. Complexe selon la revendication 1, **caractérisé en ce que** la teneur en flurane est d'au moins 5 % en poids, de préférence d'au moins 7 % en poids, plus préférablement d'au moins 8 % en poids, encore plus préférablement de 8 à 12 % en poids.

3. Complexe selon la revendication 1 ou 2, **caractérisé en ce que** le flurane est choisi parmi le groupe consistant en le sévoflurane, l'enflurane, l'isoflurane, le desflurane et le méthoxyflurane.

4. Complexe selon l'une quelconque des revendications 1 à 3, **caractérisé en ce qu'**il présente une teneur en eau de 5 à 15 % en poids, de préférence de 7 à 13 % en poids.

5. Complexe selon l'une quelconque des revendications 1 à 4 destiné à être utilisé comme médicament.

6. Anesthésique formulé pour une administration orale et/ou intraveineuse, **caractérisé en ce qu'**il comporte un complexe selon l'une quelconque des revendications 1 à 4.

7. Procédé de préparation d'un complexe selon l'une quelconque des revendications 1 à 4, **caractérisé par** les étapes suivantes :
a. préparation d'une solution aqueuse de l'α-cyclodextrine,
b. addition du flurane à la solution aqueuse,
c. séparation du complexe précipité.

8. Procédé selon la revendication 7, **caractérisé en ce que** la concentration de l'α-cyclodextrine dans la solution aqueuse préparée à l'étape a) est de 5 à 30 % en poids, de préférence de 5 à 20 % en poids, encore plus préférablement de 5 à 15 % en poids.

9. Procédé selon la revendication 7 ou 8, **caractérisé en ce que** l'addition du flurane à l'étape b) s'effectue dans un rapport molaire à l'α-cyclodextrine de 1 : 0,5 à 1 : 2, de préférence de 1 : 0,8 à 1 : 1,2.

10. Procédé selon l'une quelconque des revendications 7 à 9, **caractérisé en ce que** l'addition du flurane à l'étape b) s'effectue à la température ambiante.

11. Procédé selon l'une quelconque des revendications 7 à 10, **caractérisé en ce qu'**après l'addition du flurane s'effectue un refroidissement, de préférence à une température comprise entre 5 et 10 °C.
